(19) Europäisches Patentamt
European Patent Office
Office européen des brevets

(11) Publication number: **0 207 645**
A1

# (12) EUROPEAN PATENT APPLICATION

(21) Application number: 86304295.8

(22) Date of filing: 05.06.86

(51) Int. Cl.⁴: **C 07 C 7/148**

(30) Priority: 18.06.85 GB 8515391

(43) Date of publication of application:
07.01.87 Bulletin 87/2

(84) Designated Contracting States:
AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Denny, Patrick John
Broadmede Dalton Road Croft-on-Tees
Darlington Co Durham(GB)

(72) Inventor: Carnell, Peter John H.
50 Hartburn Village
Stockton-on-Tees Cleveland(GB)

(74) Representative: Gratwick, Christopher et al,
Imperial Chemical Industries PLC Legal Department:
Patents PO Box 6 Bessemer Road
Welwyn Garden City Herts, AL7 1HD(GB)

(54) Hydrocarbon purification.

(57) Desulphurisation of hydrocarbon streams is effected using a bed of a particulate adsorbent comprising zinc oxide at below 150°C with the hydrocarbon in the liquid state. The absorbent particles preferably have a high BET surface area and pore volume.

EP 0 207 645 A1

Hydrocarbon purification

This invention relates to hydrocarbon purification and in particular to the removal of sulphur compounds from a hydrocarbon stream.

As produced hydrocarbon streams generally contain small amounts of hydrogen sulphide and/or other sulphur compounds such as carbonyl sulphide. Before use it is generally desirable to reduce the sulphur compounds content of the hydrocarbon stream to a low level. Heretofore the sulphur compounds have generally been removed by contact of the hydrocarbon stream in the gaseous state with a bed of a particulate absorbent material, such as zinc oxide, at a relatively high temperature, often 200°C or more. We have found that such high temperatures are not necessary and that hydrocarbon streams can be desulphurised in the liquid state. This is advantageous where the hydrocarbon stream is supplied as a liquid, e.g. liquified petroleium gas or gasoline at ambient temperature, as the energy required for heating and vapourisation can be saved.

Accordingly the present invention provides a process for the removal of sulphur compounds from a hydrocarbon stream wherein the hydrocarbon stream is passed through a bed of particulate absorbent material comprising zinc oxide at a temperature on the range minus 10 to plus 150°C and at a pressure high enough to maintain a substantial proportion, preferably at least 90% by weight, of the hydrocarbon stream in the liquid state.

The hydrocarbon stream may, for example, have a boiling point at atmospheric pressure of up to 220°C, such as a naphtha or gasoline. It preferably consists essentially of hydrocarbons having two to seven carbon atoms in the molecule, such that it is gaseous, or readily volatile at atmospheric pressure without undue heating. The hydrocarbon preferably consists essentially of alkanes, or alkenes, or mixtures thereof, containing three to six carbon atoms in the molecule.

Examples of such hydrocarbon streams include natural gas liquids, associated gas liquids, petroleum processing off-gases, naphtha, and gasoline. Since the hydrocarbon stream is commonly the product of fractionating a raw gas stream containing methane and ethane, either of those hydrocarbons may be present in small quantities; likewise small quantities of nitrogen and/or carbon dioxide may be present. Also hydrogen, carbon monoxide, or alcohols such as methanol, may be present to the extent of a few percent by volume (calculated on vapour). Preferably the hydrocarbon stream is substantially dry or contains up to 5% by volume of water, calculated as vapours: in the latter event, suitable precautions should be taken to avoid deposition of ice if the processing temperature is below about 0°C.

The sulphur compounds initially present in the hydrocarbon stream usually include hydrogen sulphide and/or carbonyl sulphide, and possibly carbon disulphide, methyl mercaptan, diethyl sulphide, and/or tetramethylene sulphide. The total initial concentration of sulphur compounds, expressed as sulphur equivalent hydrogen sulphide, is typically in the range 10 to 1000 ppm by volume of the hydrocarbon stream (calculated as vapour). The absorption can be conducted so that a substantial proportion, e.g. over 75% by volume, of the sulphur content of the hydrocarbon stream can be removed. Typically the sulphur compounds content of the hydrocarbon stream leaving the absorbent bed is under 10, for example under 5, ppm by volume, expressed as above, but this is a matter of design, depending on the user's requirements.

The process is typically operated at a pressure in the range 5 to 15 bar abs. when the hydrocarbon stream is essentially butane, and 10 to 25 bar abs. when the hydrocarbon is essentially propane. In either case the temperature is typically in the range 20 to 60°C. Atmospheric pressure is suitable for normally liquid hydrocarbon streams, such as hexane or gasoline.

The absorbent material preferably comprises at least 60, especially at least 80, % by weight of zinc oxide, calculated on the absorbent material non-volatile at 900°C. As used in the process the zinc oxide may be, at least initially, wholly or partly hydrated or in the form of a salt of a weak acid, e.g. a carbonate.

The absorbent material is preferably in the form of porous agglomerates, as may be made, for example, by mixing a finely divided zinc oxide composition with a cement binder and a little water, insufficient to give a slurry, and then granulated or extruded. In order to aid access of the hydrocarbon stream into the prticles, the latter may be provided in the form of extruded pellets having a plurality of through passages. Typically the BET surface area of the particles is at least 20, preferably in the range 50 to 200, $m^2.g^{-1}$, and the pore volume of the particles is preferably at least 0.2 $cm^3.g^{-1}$.

Since the absorption efficiency and hence the life of a zinc oxide particulate bed depends on the rate of diffusion of the zinc sulphide formed by reaction of the zinc oxide with the sulphur compounds towards the interior of the particle at such low absorption temperatures, it is preferably to employ zinc oxide particles having a high pore volume, above 0.2 $cm^3.g^{-1}$ and high surface area, above 50 $m^2.g^{-1}$. Thus while zinc oxide particles having a lower pore volume and a surface area of the order of 25 to 30 $m^2.g^{-1}$ can be employed, the bed life at low absorption temperatures is relatively low, necessitating he use of large bed volumes to avoid premature break-through of the sulphur compounds into the exit hydrocarbon stream. By using a bed of particles of pore volume above, for example, 0.25 $cm^3.g^{-1}$ and surface area above, for example, 70 $m^2.g^{-1}$, the bed volume can be markedly reduced, e.g. to about one third of that required with particlesof low pore volume and surface area 25 to 30 $m^2.g^{-1}$. The particles employed thus preferably have a surface area above 50, particularly above 70, $m^2.g^{-1}$ and

a pore volume above 0.25 $cm^3.g^{-1}$.

Preferred absorbent materials for the process have a hydrogen sulphide absorption capacity of at least 20, especially at least 25, % of the theoretical, at a temperature of 25°C, as determined in a standard test in which a mixture of hydrogen sulphide (2000 ppm by volume), carbon dioxide (4% by volume), and methane (balance) is passed through a bed of the particles at atmospheric pressure and a space velocity of 700 $h^{-1}$ using a bed of circular cross section having a length to diameter ratio of 5.

A particularly suitable particulate zinc oxide material is that sold by Imperial Chemical Industries plc as "Catalyst 75-1". These particles are granules typically having a surface area of the order of 80 $m^2.g^{-1}$ and a pore volume of about 0.3 $cm^3.g^{-1}$, and an adsorption capacity of about 27% of theoretical when measured by the above procedure.

The particulate bed can be in the form of a fixed bed, a liftable bed, or a fluidised bed.

The process can be the sole sulphur removal step applied to the hydrocarbon stream or can be used in combination with one or more of the following:

upstream distillative removal of hydrogen sulphide;

upstream removal of sulphur compounds and/or carbon dioxidein an absorbent liquid such as an ethanolamine, "Sulpholane", or methanol;

upstream hydrogenative conversion of organic sulphur compounds to hydrogen sulphide;

downstream removal of carbonyl sulphide, carbon disulphide, methyl mercaptan and/or residual hydrogen sulphide by reaction with caustic alkali or soda-lime, possibly in the presence of an alcohol.

In a preferred process a normally liquid hydrocarbon is vaporised and, after addition of hydrogen, is passed through a bed of a hydrodesulphurisation catalyst, e.g. cobalt molybdate, at elevated temperature, e.g. 350 to 400°C, to convert organic sulphur compounds to hydrogen sulphide. The resultant mixture is cooled and the gaseous components, viz hydrogen sulphide and any excess of hydrogen, are flashed off. The resulting liquid is then treated in accordance with the present invention in order to reduce the residual hydrogen sulphide content to an acceptable level.

A specific process of operating the invention using as the hydrocarbon feedstock a water-free liquid propane stream produced by fractionating associated gas at a North Sea oilfield and containing 2 ppm by volume of hydrogen sulphide involves passing the hydrocarbon stream at a rate of about 2500 te per day through a fixed bed of volume 50 $m^3$ (about 40 te) of ICI "Catalyst 75-1" granules having a diameter in the range 0.5 to 5 mm, a BET surface area of about 80 $m^2.g^{-1}$ and a pore volume of 0.3 $cm^3.g^{-1}$ at a pressure of 20 bar abs. and a temperature of 45°C.

Another specific process using a water-free liquid butane stream from the same source and containing 2 ppm by volume of hydrogen sulphide involves passing the hydrocarbon stream through a similar zinc oxide bed at the same rate, but at a pressure of 8 bar abs. and a temperature of 50°C.

In each such operation it is to be expected that an outlet hydrogen concentration well under 0.5 ppm by volume will be maintained for at least 1 year.

The invention is illustrated by the following examples.

### Example 1

A column of diameter 2.5 cm was charged with 60 g of "Catalyst 75-1" granules of size 3 to 5 mm to form a bed of length 12 cm. Propylene, containing 263 ppm of carbonyl sulphide and 2 ppm of water, both by weight, at a pressure of about 15 bar abs was passed at ambient temperature (which varied during the course of the experiment between -2°C and +10°C) down through the bed at a rate of 60 g.hr$^{-1}$. The carbonyl sulphide content of the stream leaving the bed was monitored and remained below 20 ppm by weight until the liquid propylene had been flowing through the bed for over 220 hours whereupon the carbonyl sulphide content of the effluent stream rose rapidly.

## Example 2

The procedure of Example 1 was repeated using as the feedstock a stream of n-hexane containing 1600 ppm by volume (expressed as vapours) of hydrogen sulphide at atmospheric pressure, a temperature of $50^\circ$C and a liquid space velocity of 2 $hr^{-1}$. In this case the "Catalyst 75-1" granules had a size of 1.7 to 2.1 mm. No hydrogen sulphide was detected in the effluent and the experiment was discontinued after 380 min. Analysis of the bed showed that the top one-sixth of the bed had a sulphur content of 9.4% by weight and about 94% by weight of the absorbed sulphur was in the top half of the bed.

## Example 3

Example 2 was repeated using as the feedstock a stream of n-butane containing 350 ppm (increased to 600 ppm during the course of the experiment) by volume (expressed as vapours) of hydrogen sulphide, a pressure of 10 bar abs., a temperature of $30^\circ$C and a liquid space velocity of 4 $hr^{-1}$. No hydrogen sulphide was detected in the effluent until break-through occurred after a total flow time of 89 hours. The top one sixth of the bed had a sulphur content of 20.4% by weight and about 80% by weight of the absorbed sulphur was in the top half of the bed.

PA/CG/MP

29 May 1986/L162

1.     A process for the removal of sulphur compounds from a hydrocarbon stream wherein the hydrocarbon stream is passed through a bed of particulate absorbent material comprising zinc oxide at a temperature on the range minus 10 to plus 150°C and at a pressure high enough to maintain a substantial proportion of the hydrocarbon stream in the liquid state.

2.     A process according to claim 1 wherein the hydrocarbon stream consists essentially of hydrocarbons having two to seven carbon atoms in the molecule.

3.     A process according to claim 2 wherein the hydrocarbon stream consists essentially of butane and is at a pressure of 5 to 15 bar abs. and at a temperature of 20 to 60°C.

4.     A process according to claim 2 wherein the hydrocarbon stream consists essentially of propane and is at a pressure of 10 to 25 bar abs. and at a temperature of 20 to 60°C.

5.     A process according to any one of claims 1 to 4 wherein the absorbent material comprises at least 80% by weight of zinc oxide, calculated on the constituents non-volatile at 900°C.

6.     A process according to any one of claims 1 to 5 wherein the absorbent material has a BET surface area in the range 50 to 200 $m^2.g^{-1}$ and a pore volume of at least 0.2 $cm^3.g^{-1}$.

7.     A process according to claim 6 wherein the absorbent material has a BET surface area of at least 70 $m^2.g^{-1}$ and a pore volume of at least 0.25 $cm^2.g^{-1}$.

8.     A process according to any one of claims 1 to 7 wherein the absorbent material has a hydrogen sulphide absorption capacity of at least 25% of theoretical at 25°C.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-1 142 339 (BASF) <br> * Page 2 * | 1,2,5 | C 07 C 7/148 |
| X | EP-A-0 016 284 (DAVY McKEE (OIL & CHEMICALS)) <br> * Claims * | 1,2,5 | |
| P,X | EP-A-0 169 828 (LABOFINA) <br><br> * Claims * | 1,2,5-7 | |

**TECHNICAL FIELDS SEARCHED (Int. Cl.4)**

C 07 C 7/00
C 10 G 29/00

The present search report has been drawn up for all claims

| Place of search | Date of completion of the search | Examiner |
|---|---|---|
| THE HAGUE | 25-09-1986 | VAN GEYT J.J.A. |